# EUROPÄISCHE PATENTANMELDUNG

(11) **EP 4 098 307 A1**
(43) Veröffentlichungstag der Anmeldung: **07.12.2022**
(21) Anmeldenummer: 22000147.3
(22) Anmeldetag: 02.06.2022
(51) Int. Cl.: A61M 16/10, A61L 9/18

(54) **VORRICHTUNG UND VERFAHREN ZUR HERSTELLUNG UND BEREITSTELLUNG VON SINGULETT-SAUERSTOFF IM THERAPIEVERFAHREN**

(30) Priorität: 03.06.2021 DE 102021002837
(71) Anmelder: Hellstern Stiftung, 9495 Triesen (LI)
(72) Erfinder: BAYER, Meik, 08058 Zwickau (DE)
(74) Vertreter: Auerbach, Bettina

(57) **Zusammenfassung**

Die Erfindung betrifft eine Vorrichtung zur Herstellung und Bereitstellung von Singulett-Sauerstoff im Therapieverfahren, bestehend aus einem Lufteinlasskanal, einem HEPA-Filter, einer Aktivkammer, verbindenden Luftführungskanälen und einem Luftauslasskanal, wobei der Lufteinlasskanal (1) direkt mit einem HEPA-Filter (2) verbunden ist, der HEPA-Filter (2) über einen Luftführungskanal (8) mit einem UV-Tunnel (3) verbunden angeordnet ist, über mehrere Luftführungskanäle (8) zur Weiterleitung der entkeimten Luft aus dem UV-Tunnel (3) ist dieser mit einer Befeuchtungskammer (9) für die Luft verbunden, welche wiederum mit Ihrem ausgangsseitigen Luftführungskanal mit dem, mehrere Aktiv-Kammern aufweisenden Singulett-Sauerstoff-Aktivator (4) verbunden ist. Über wenigstens einen Luftführungskanal (8) ist der Singulett-Sauerstoff-Aktivator (4) mit einer nachgeschalteten Wirkstoffkammer (5) verbunden, wobei an dieser Wirkstoffkammer (5) mit darin angeordnetem Wirkstoffreservoir die Luftableitung (6) angeordnet ist, an welcher die Atemmaske (7) angeordnet ist. Zur regulierten Ansaugung von Atemluft ist eine Ansaugpumpe mit einer Steuerelektronik angeordnet.

## Beschreibung

Die Erfindung betrifft eine Vorrichtung und ein Verfahren zur Herstellung und Bereitstellung von durch den menschlichen Körper verwertbarem Singulett-Sauerstoff für die Anwendung in Therapieverfahren zur Verbesserung und Erhöhung der Sauerstoffaufnahme durch den menschlichen Körper.

Ähnliche Verfahren dieser Art sind bereist bekannt. So beschreibt die DE 100 48 153 A1 eine Vorrichtung zur Erzeugung von Singulett-Sauerstoff sowie ein Verfahren zur Herstellung einer mit Farbstoff beschichteten Fläche. Bei der Vorrichtung von Singulett-Sauerstoff liegen sich zwei Flächen innerhalb eines Gehäuses gegenüber. Die eine Fläche ist speziell mit einem Farbstoff beschichtet. Die andere Scheibe ist lichtdurchlässig und filtert aus der dahinter angeordneten Lichtquelle Lichtwellenlängen von 600 bis 680 nm, Konkret im Ausführungsbeispiel werden Lichtwellenlängen von 634,3 nm durch die lichtdurchlässige Scheibe herausgefiltert, so dass eine Strahlung mit einem bestimmten Frequenzspektrum herausgefiltert wird. Der Farbstoff der gegenüberliegenden Fläche wird angeregt. Der Sauerstoff aus der vorbeiströmenden Luft wird durch die Beeinflussung der Lichteffekte im Tunnel zwischen den beiden Scheiben angeregt und wandelt sich in Singulett-Sauerstoff um. Um die Eigenschaften des Farbstoffs nicht zu beeinträchtigen, wird diese mit Farbstoff beschichtete Fläche durch ein Substrat mit einer fein aufgerauten Fläche gebildet und der Farbstoff wird in die Vertiefungen der fein angerauten Fläche hinein poliert. Da die Lichtquelle aus LEDs erst durch eine Glasscheibe hindurch geleitet wird um hierdurch eine Eliminierung der Strahlungsspektren zu erhalten, wird die Beeinflussung des Farbstoffes an das Absorptionsmaximum angepasst.

Diese Art der Ausfilterung bzw. Selektierung von Lichtemissionswellenlängen ist sehr aufwendig und kompliziert, da nur ganz spezielles Glas und dieses jeweils auf den eingesetzten Farbstoff abgestimmt, eingesetzt werden kann. Dadurch erhöhen sich die Kosten der Vorrichtung. Ein weiterer Nachteil ist dabei, dass der Singulett-Sauerstoff nicht stabil ist und immer bestrebt ist, sofort wieder eine Bindung einzugehen.

Es ist deshalb Aufgabe der Erfindung, eine Lösung zu finden, welche die Nachteile des Standes der Technik überwindet und effektiv sowie kostengünstig hergestellt werden kann. Vor allem soll mit der Vorrichtung erreicht werden, dass keine Vieren oder andere Verunreinigungen der Luft in das System eindringen und vom Körper letztendlich aufgenommen werden können und dass die Aufnahme des Sauerstoffs vom menschlichen Körper effizient verbessert werden kann. Es soll ein Transportweg geschaffen werden, um energiereiche Wassermoleküle zur Anregung der Mitochondrien in den Körper mit zu transportieren. Angestrebt werden soll auch die Schaffung von kurzen Transportwegen.

Erfindungsgemäß wir die Aufgabe durch die Merkmale der Patentansprüche 1 und 7 gelöst, wobei die vorteilhaften Ausgestaltungen in den dazugehörigen Unteransprüchen beschrieben sind.

Danach besteht die Vorrichtung zur Herstellung und Bereitstellung von durch Singulett-Sauerstoff energetisch angereichertem Wassermolekülen im Therapieverfahren zunächst aus einem Lufteinlasskanal, einem HEPA-Filter, einer Aktivkammer, verbindenden Luftführungskanälen und einem Luftauslasskanal.

Erfindungsgemäß ist der Lufteinlasskanal direkt mit einem HEPA-Filter verbunden, wo die Reinigung der angesaugten Luft von Verunreinigungen, wie Viren, Milbeneier, Pollen, Aerosole, Keime und weitere auch toxischen Staubpartikel aus der Luft ausgefiltert werden.

Der HEPA-Filter wiederum ist über einen Luftführungskanal mit einem UV-Tunnel verbunden. In diesem UV-Tunnel werden die in der Raumluft schädlichen Schimmelpilze, Viren, Bakterien und Hefen deaktiviert. Diese Kombination sorgt dafür, dass über die Luft während der Therapie keine Verunreinigungen mehr in den Körper gelangen.

Der UV-Tunnel ist über mehrere Luftführungskanäle mit einer Befeuchtungskammer konstruktiv verbunden. Durch wird die angesogene gereinigte und entkeimte Luft befeuchtet. Über einen ausgangsseitigen Luftführungskanal ist der UV-Tunnel mit der anschließend angeordneten Befeuchtungskammer zur Weiterleitung der entkeimten und befeuchteten Luft mit dem, mehrere Aktiv-Kammern aufweisenden Singulett-Sauerstoff-Aktivator verbunden. In diesem Singulett-Sauerstoff-Aktivator ist eine Lochscheibe mit einer integrierter LED-Lichtquelle und eine zu dieser Scheibe im Abstand gegenüberliegend angeordnete Scheibe mit einer durch Licht anregbaren Mehrfach-Farbbeschichtung angeordnet. Diese Farbbeschichtung ist so gewählt, dass der Sauerstoff in Singulett-Sauerstoff übergeht. Die Scheiben sind mit ihren Randbereichen im inneren Gehäuserand so befestigt und angeordnet, dass sie eine Kammer bilden, wobei die zwischen den Scheiben gebildete mögliche Durchführung für die Luft eingangs- und ausgangseitig jeweils mit einer Durchführungsöffnung für die Anordnung eines Luftführungskanals für die Luftzuführung und für die Luftabführung ausgestattet ist. Dieser Luftführungskanal verbindet den Singulett-Sauerstoff-Aktivator mit einer nachgeschalteten Wirkstoffkammer. In der Wirkstoffkammer ist ein von der gereinigten und mit durch Singulett-Sauerstoff energetisch angereichertem Wassermolekülen aufbereitete Luft durchströmbares und austauschbares Wirkstoffreservoir angeordnet. Im Wirkstoffreservoir können als Wirkstoff Propolis, OPC und/oder Resveratrol angeordnet sein, um die Atemwege zusätzlich für die Aufnahme der durch Singulett-Sauerstoff energetisch angereichertem Wassermolekülen aufbereitete Luft vorzubereiten und zu unterstützen.

An dieser Wirkstoffkammer mit darin angeordnetem Wirkstoffreservoir ist die Luftableitung angeordnet, an welcher die Atemmaske angeordnet ist, wobei zur regulierten Ansaugung von Atemluft eine Ansaugpumpe mit einer Steuerelektronik angeordnet ist.

Vorteilhafter Weise und zur Verkürzung der Transportwege der Luft sind der HEPA-Filter und der UV-Tunnel mit der Befeuchtungskammer in einer Baueinheit angeordnet.

Eine weitere vorteilhafte Ausgestaltung sieht vor, dass der Singulett-Sauerstoff-Aktivator aus einem Gehäuse besteht, in dem wenigsten 2 Kammern angeordnet sind, welche jeweils über eine Scheibe mit integrierter LED-Lichtquelle und einer zu dieser Scheibe im Abstand gegenüberliegend angeordneten Scheibe mit einer durch Licht anregbaren Farbbeschichtung verfügt, wobei die Scheiben mit den Randbereichen im inneren Gehäuserand befestigt angeordnet sind und die Kammerbildung allein durch die Scheibenanordnung realisiert ist und jeder Kammer im Gehäuse jeweils eine Durchführungsöffnung für die Anordnung der Luftführungskanäle (8) für die Luftzuführung und für die Luftabführung zugeordnet ist. Diese mehreren Kammern führen zur Verkürzung der Transportwege, da der Luftstrom geteilt und in Teilströmen in den einzelnen Kammern aufbereitet wird.

Um eine hohe Effizienz der durch Singulett-Sauerstoff energetisch angereicherten Wassermoleküle zu erreichen ist es wichtig, dass die Farbschicht auf der Scheibe 100-prozentig ausgeführt ist, um die optimale Absorption der gegenüberliegenden Lichtquelle zu erreichen und so den dazwischen befindlichen Sauerstoff in der gereinigten und mit Feuchtigkeit beaufschlagten Trägerluft in den Singulett-Sauerstoff-Zustand zu überführen. Dazu weist die Scheibe wenigstens zwei nacheinander aufgetragene Farbschichten auf. Es handelt sich um ein spezielles rotes Licht, das benötigt wird, um Singulett-Sauerstoff zu bilden.

Es hat sich als Vorteil herausgestellt, dass die Scheibe mit der intergierte LED-Lichtquelle als Lochscheibe ausgebildet ist, wobei an jedem Loch ein LED angeordnet ist, welches jeweils in Richtung farbstoffbeschichteter Scheibe in einem Lichtwellenbereich von 630 nm bis 640 nm strahlt. Weiterhin ist vorteilhaft, dass die Lichtemission der auf der Lochscheibe angeordneten LED-Lichtquellen einen Wellenlängenbereich von 630 - 640 nm aufweist, wobei die Farbstoffbeschichtung auf der gegenüberliegenden Scheibe genau in diesem Bereich ihre absolut höchste Absorption erreicht.

Die erfinderische Lösung basiert auch auf einem Verfahren zur Herstellung und Bereitstellung von Singulett-Sauerstoff für Therapiezwecke, bei dem zunächst mit dem Einsatz eines HEPA-Filters und eines nachgeschalteten UV-Tunnels eine doppelte und sichere Entkeimung und damit Absonderung von Viren, Milbeneiern, Pollen, Aerosolen, krankheitserregenden Keimen und weiteren toxischen Staubpartikel der angesaugten Luft durchgeführt wird

Anschließend erfolgt in einer Befeuchtungskammer, die Befeuchtung der entkeimten und gereinigten Luft, um die angesaugte und zu behandelnde Luft einem mehrere Aktivkammern aufweisenden Singulett-Sauerstoff-Aktivator (4) zuzuführen. Dort wird der Sauerstoff der Luft in Singulett-Sauerstoff überführt, in dem mittels der angeordneten Lichtquellen, die im Frequenzspektrum von 630 nm bis 640 nm auf die gegenüberliegende mit rotem Farbstoff mehrfach beschichtete Fläche einwirken. Diese Farbstoffoberfläche absorbiert das Licht entsprechend und der zwischen den beiden Scheiben durchströmende Sauerstoff der Luft dabei so aktiviert und anregt, dass sich dieser in Singulett-Sauerstoff umwandelt und die Wassermoleküle der befeuchteten Luft energetisch anreichert. Dies ist notwendig, da der Singulett-Sauerstoff -Zustand nur kurze Zeit gehalten werden kann, da er stark bestrebt ist, sich mit anderen Elementen chemisch zu verbinden. Diese Energie wird in den Wassermolekülen gespeichert und gelangt so in den menschlichen Körper und führt zur Anreicherung der menschlichen Zellen mit Energie.

Diese energetische Anregung erfolgt dabei in mehreren parallel arbeitenden Aktivkammern.

Nach dem Verlassen der mit Singulett-Sauerstoff angeregten Luft wird diese durch eine Wirkstoffkammer mit einem oder mehreren Wirkstoffreservoirs geführt, wo die Luft mit dem jeweiligen Wirkstoff zur weiteren positiven Beeinflussung der Sauerstoff-Aufnahme der Atemwege und -organe und zur Verbesserung dieser angereichert wird. Über das Mundstück am Ende der Luftableitung gelangt die aufbereitete und energetisch angereicherte Luft auf kurzem Weg zum Verbraucher.

Die Erfindung soll anhand eines Ausführungsbeispiels näher erläutert werden.

Dazu zeigen
- Fig. 1: eine schematische Darstellung einer Vorrichtung zur Herstellung und Bereitstellung von Singulett-Sauerstoff für die Anwendung in Therapieverfahren und
- Fig. 2: eine schematische Schnittdarstellung durch den Aktivator 4 mit seinen die einzelnen Aktivkammern bildenden Flächen 4.1 und 4.2.

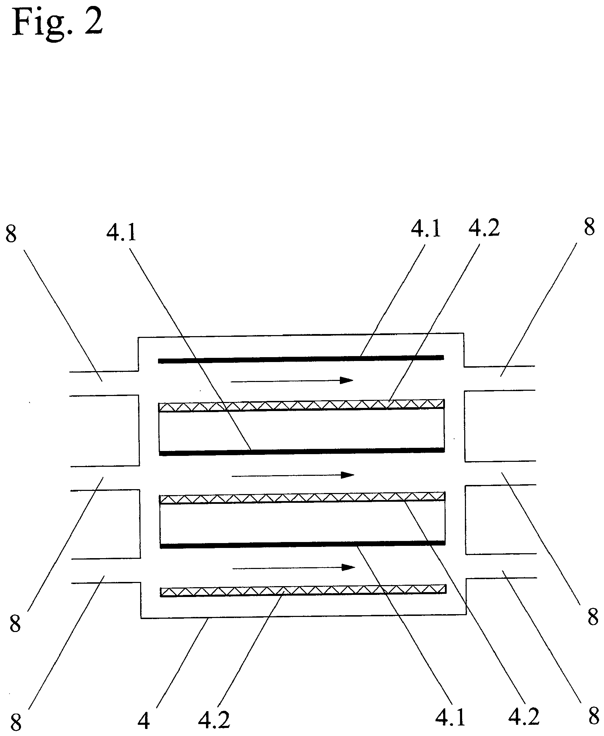

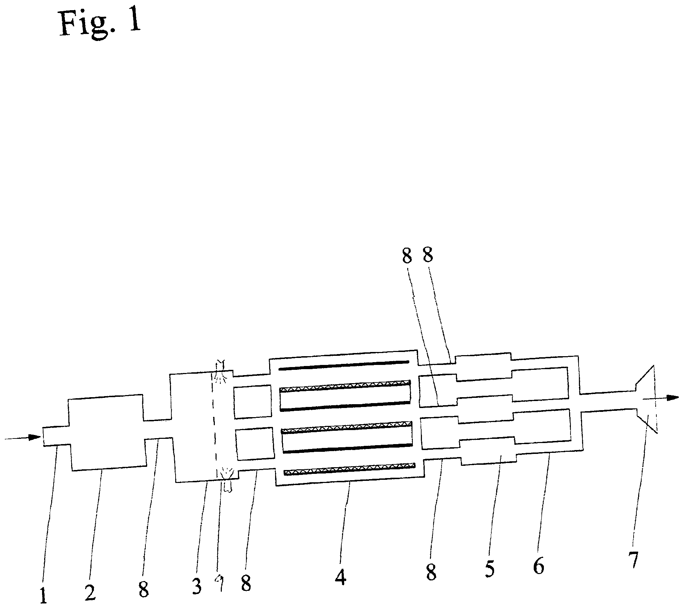

Gemäß der Figuren 1 und 2 besteht eine Vorrichtung zur Herstellung und Bereitstellung von Singulett-Sauerstoff im Therapieverfahren aus einem Lufteinlasskanal, einem HEPA-Filter, einer Aktivkammer, verbindenden Luftführungskanälen und einem Luftauslasskanal. Die erfindungsgemäße Vorrichtung sieht vor, dass der Lufteinlasskanal 1 direkt mit einem HEPA-Filter 2 verbunden ist und dieser HEPA-Filter 2 über einen Luftführungskanal 8 mit einem UV-Tunnel 3 in Verbindung steht. Im HEPA Filter 2 erfolgt die Reinigung der angesaugten Luft von Verunreinigungen, wie Viren, Milbeneier, Pollen, Aerosole, Keime und weitere auch toxischen Staubpartikel.

Im UV-Tunnel werden die in der Raumluft schädlichen Schimmelpilze, Viren, Bakterien und Hefen deaktiviert. Diese doppelte Reinigung der Luft ist wichtig, um während der Therapie die Atemwege zu schonen und effizient für die Aufnahme der energetisch angereicherten Luft zu unterstützen.

Der UV-Tunnel 3 ist mit einer Befeuchtungskammer 9 für die Luft verbunden. Diese ist ausgangsseitig über einen Luftführungskanal mit dem Singulett-Sauerstoff-Aktivator 4 verbunden. Der Singulett-Sauerstoff-Aktivator verfügt über mehrere Aktiv-Kammern. Über wenigstens einen Luftführungskanal 8 ist der Singulett-Sauerstoff-Aktivator 4 mit einer nachgeschalteten Wirkstoffkammer 5 verbunden. In der Wirkstoffkammer 5 sind wenigstens ein Wirkstoffreservoir angeordnet, mit dem die bereits energetisch angereicherte Luft zusätzlich mit, die Aufnahme der Energie fördernden Wirkstoffen, wie Propolis, OPC und/oder Resveratrol angereichert wird.

An der Wirkstoffkammer 5 ist die Luftableitung 6 angeordnet, an welcher die Atemmaske 7 für den Therapiepatienten angeordnet ist.

Zur regulierten Ansaugung von Atemluft ist eine Ansaugpumpe mit einer Steuerelektronik in der Vorrichtung integriert.

Der HEPA-Filter2 und der UV-Tunnel 3 mit der Befeuchtungskammer 9 sind in einer Baueinheit angeordnet, um die Transportwege der angesaugten und aufbereiteten Luft so kurz wie möglich zu gestalten. Aus der Befeuchtungskammer 9 wird die gereinigte und aufbereitete Luft über mehrere Luftführungskanäle 8 zum Singulett-Sauerstoff-Aktivator 4 transportiert. Dieser Singulett-Sauerstoff-Aktivator 4 besteht aus einem Gehäuse. In diesem sind wenigsten 2 Kammern angeordnet, welche jeweils über eine Scheibe 4.2 mit integrierter LED-Lichtquelle und einer zu dieser Scheibe im Abstand gegenüberliegend angeordneten Scheibe 4.1 mit einer durch Licht anregbaren roten Farbbeschichtung verfügt, wobei die Scheiben mit den Randbereichen im inneren Gehäuserand befestigt angeordnet sind und die Kammerbildung allein durch die Scheibenanordnung realisiert ist und jeder Kammer im Gehäuse jeweils eine Durchführungsöffnung für die Anordnung der Luftführungskanäle 8 für die Luftzuführung und für die Luftabführung zugeordnet ist.

Die Farbbeschichtung der Scheibe 4.1 besteht aus 3 nacheinander aufgetragenen roten Farbschichten.

Die Scheibe 4.2 mit integrierter LED-Lichtquelle ist als Lochscheibe ausgebildet, wobei an jedem Loch ein LED angeordnet ist, welches jeweils in Richtung farbstoffbeschichteter Scheibe 4.1 in einem Lichtwellenbereich von 630 nm bis 640 nm strahlt. Dabei weist die Lichtemission der auf der Lochscheibe 4.2 angeordneten LED-Lichtquellen einen Wellenlängenbereich von 630 - 640 nm auf, was für die Farbstoffbeschichtung auf der gegenüberliegenden Scheibe 4.1 bedeutet, dass diese genau in diesem Bereich ihre absolut höchste Absorption erreicht.

In der nachfolgend angeordneten Wirkstoffkammer 5 sind drei von der Luft durchströmbare und austauschbare Wirkstoffreservoirs angeordnet. Ein Wirkstoffreservoir beinhaltet Propolis, das zweite beinhaltet OPC (oligomere Procyanidine aus Traubenkernextrakt) und das dritte Reservoir beinhaltet Resveratrol.

Zunächst wird mit dem Einsatz eines HEPA-Filters 2 und des nachgeschalteten UV-Tunnels 3 eine doppelte und sichere Entkeimung und damit Absonderung von Viren, Milbeneiern, Pollen, Aerosolen, krankheitserregenden Keimen und weiteren toxischen Staubpartikel der angesaugten Luft durchgeführt wird. Anschließend wird die gereinigte Luft einer Befeuchtungskammer zugeführt, wo diese Luft befeuchte wird.

Diese befeuchtete Luft wird nunmehr einem mehrere Aktivkammern aufweisenden Singulett-Sauerstoff-Aktivator 4 zugeführt, wo der Sauerstoff der Luft in Singulett-Sauerstoff überführt wird, in dem mittels der angeordneten Lichtquellen 4.2, die im Frequenzspektrum von 630 nm bis 640 nm auf die gegenüberliegende mit Farbstoff dreifach beschichtete Fläche 4.1 einwirken, diese Farbstoffoberfläche das Licht entsprechend absorbiert und der zwischen den beiden Scheiben 4.1, 4.2 durchströmende Sauerstoff der Luft dabei so aktiviert und anregt wird, dass sich dieser in Singulett-Sauerstoff umwandelt. Diese Anregung erfolgt dabei in drei parallel arbeitenden Aktivkammern. Nach dem Verlassen der mit Singulett-Sauerstoff angeregten Luft wird diese durch eine Wirkstoffkammer mit drei (Propolis, OPC und Resveratrol) Wirkstoffreservoirs geführt, wo die Luft mit dem jeweiligen Wirkstoff zur weiteren positiven Beeinflussung der Sauerstoff-Aufnahme der Atemwege und - organe und zur Verbesserung dieser angereichert wird und gelangt über das Mundstück am Ende der Luftableitung 6 auf kurzem Weg zum Verbraucher.

Die Vorteile der Erfindung liegen zusammengefasst in der Mehrfachentkeimung und Reinigung der angesaugten Luft, der Anreicherung mit heilenden und die Sauerstoffaufnahme fördernden Wirkstoffen, in der Schaffung on kurzen Transportwegen durch das örtliche Verbinden von Aufbereitungstools und in der effizienten Gestaltung der Aktivkammer durch Mehrfachbeschichtung und der Zuordnung einer Befeuchtung der Luft, um die Energie bei der Bildung des Singulett-Sauerstoffs aufzunehmen und bis zum Verbraucher auf kurzem Weg transportieren zu können.

### Bezugszeichenliste

- 1: Lufteinlasskanal
- 2: HEPA-Filter
- 3: UV-Tunnel
- 4: Singulett-Sauerstoff-Aktivator
- 4.1: Scheibe mit einer durch Licht anregbaren Farbbeschichtung
- 4.2: Lochscheibe mit integrierter LED-Lichtquelle
- 5: Wirkstoffkammer
- 6: Luftableitung
- 7: Atemmaske
- 8: Luftführungskanal
- 9: Befeuchtungskammer

## Patentansprüche

1. Vorrichtung zur Herstellung und Bereitstellung von Singulett-Sauerstoff im Therapieverfahren, bestehend aus einem Lufteinlasskanal, einem HEPA-Filter, einer Aktivkammer, verbindenden Luftführungskanälen und einem Luftauslasskanal,
**dadurch gekennzeichnet, dass**
- der Lufteinlasskanal (1) direkt mit einem HEPA-Filter (2) verbunden ist,
- der HEPA-Filter (2) über einen Luftführungskanal (8) mit einem UV-Tunnel (3) verbunden angeordnet ist,
- über mehrere Luftführungskanäle (8) zur Weiterleitung der entkeimten Luft aus dem UV-Tunnel (3) ist dieser mit einer Befeuchtungskammer (9) für die Luft verbunden, welche wiederum mit Ihrem ausgangsseitigen Luftführungskanal mit dem, mehrere Aktiv-Kammern aufweisenden Singulett-Sauerstoff-Aktivator (4) verbunden ist,
- über wenigstens einen Luftführungskanal (8) ist der Singulett-Sauerstoff-Aktivator (4) mit einer nachgeschalteten Wirkstoffkammer (5) verbunden,
- an dieser Wirkstoffkammer (5) mit darin angeordnetem Wirkstoffreservoir ist die Luftableitung (6) angeordnet, an welcher die Atemmaske (7) angeordnet ist,
- wobei zur regulierten Ansaugung von Atemluft eine Ansaugpumpe mit einer Steuerelektronik angeordnet ist.

2. Vorrichtung zur Herstellung und Bereitstellung von Singulett-Sauerstoff nach dem Patentanspruch 1, **dadurch gekennzeichnet, dass** der HEPA-Filter (2) und der UV-Tunnel (3) mit der Befeuchtungskammer (9) in einer Baueinheit angeordnet sind.

3. Vorrichtung zur Herstellung und Bereitstellung von Singulett-Sauerstoff nach einem der Ansprüche 1 und 2, **dadurch gekennzeichnet, dass** der Singulett-Sauerstoff-Aktivator (4) aus einem Gehäuse besteht, in dem wenigsten 2 Kammern angeordnet sind, welche über jeweils eine Scheibe (4.2) mit intergierter LED-Lichtquelle und einer zu dieser Scheibe im Abstand gegenüberliegend angeordneten Scheibe (4.1) mit einer durch Licht anregbaren Farbbeschichtung verfügt, wobei die Scheiben mit den Randbereichen im inneren Gehäuserand befestigt angeordnet sind und die Kammerbildung allein durch die Scheibenanordnung realisiert ist und jeder Kammer im Gehäuse jeweils eine Durchführungsöffnung für die Anordnung der Luftführungskanäle (8) für die Luftzuführung und für die Luftabführung zugeordnet ist.

4. Vorrichtung zur Herstellung und Bereitstellung von Singulett-Sauerstoff nach einem der Ansprüche 1 bis 3, **dadurch gekennzeichnet, dass** die Farbbeschichtung der Scheibe (4.1) aus mindestens 2 nacheinander aufgetragenen Farbschichten besteht.

5. Vorrichtung zur Herstellung und Bereitstellung von Singulett-Sauerstoff nach einem der Ansprüche 1 bis 4, **dadurch gekennzeichnet, dass** die Scheibe (4.2) mit intergierter LED-Lichtquelle als Lochscheibe ausgebildet ist, wobei an jedem Loch ein LED angeordnet ist, welches jeweils in Richtung farbstoffbeschichteter Scheibe (4.1) in einem Lichtwellenbereich von 630 nm bis 640 nm strahlt.

6. Vorrichtung zur Herstellung und Bereitstellung von Singulett-Sauerstoff nach einem der Ansprüche 1 bis 5, **dadurch gekennzeichnet, dass** die Lichtemission der auf der Lochscheibe (4.2) angeordneten LED-Lichtquellen einen Wellenlängenbereich von 630 - 640 nm aufweist, wobei die Farbstoffbeschichtung auf der gegenüberliegenden Scheibe (4.1) genau in diesem Bereich ihre absolut höchste Absorption erreicht.

7. Vorrichtung zur Herstellung und Bereitstellung von Singulett-Sauerstoff nach einem der Ansprüche 1 bis 6, **dadurch gekennzeichnet, dass** in der Wirkstoffkammer (5) ein von der Luft durchströmbares und austauschbares Wirkstoffreservoir angeordnet ist.

8. Vorrichtung zur Herstellung und Bereitstellung von Singulett-Sauerstoff nach einem dem Anspruch 7, **dadurch gekennzeichnet, dass** der HEPA-Filter (2) und der UV-Tunnel (3) in einer Arbeitsvorrichtung angeordnet sind.

9. Vorrichtung zur Herstellung und Bereitstellung von Singulett-Sauerstoff nach einem dem Anspruch 7, **dadurch gekennzeichnet, dass** im Wirkstoffreservoir der Wirkstoffkammer (5) als Wirkstoff Propolis, OPC und/oder Resveratrol angeordnet ist.

10. Verfahren zur Herstellung und Bereitstellung von Singulett-Sauerstoff nach einem der Ansprüche 1 bis 8, **dadurch gekennzeichnet, dass**
- zunächst mit dem Einsatz eines HEPA-Filters (2) und eines nachgeschalteten UV-Tunnels (3) eine doppelte und sichere Entkeimung und damit Absonderung von Viren, Milbeneiern, Pollen, Aerosolen, krankheitserregenden Keimen und weiteren toxischen Staubpartikel der angesaugten Luft durchgeführt wird,
- anschließend wird die angesaugte und zu behandelnde Luft einem mehrere Aktivkammern aufweisenden Aktivator (4) zugeführt, wo der Sauerstoff der Luft in Singulett-Sauerstoff überführt wird, in dem mittels der angeordneten Lichtquellen (4.2), die im Frequenzspektrum von 630 nm bis 640 nm auf die gegenüberliegende mit Farbstoff mehrfach beschichtete Fläche (4.1) einwirken, diese Farbstoffoberfläche das Licht entsprechend absorbiert und der zwischen den beiden Scheiben (4.1, 4.2) durchströmende Sauerstoff der Luft dabei so aktiviert und anregt wird, dass sich dieser in Singulett-Sauerstoff umwandelt,
- diese Anregung erfolgt dabei in mehreren parallel arbeitenden Aktivkammern,
- nach dem Verlassen der mit Singulett-Sauerstoff angeregten Luft wird diese durch eine Wirkstoffkammer mit einem oder mehreren Wirkstoffreservoirs geführt, wo die Luft mit dem jeweiligen Wirkstoff zur weiteren positiven Beeinflussung der Sauerstoff-Aufnahme der Atemwege und -organe und zur Verbesserung dieser angereichert wird und gelangt über das Mundstück am Ende der Luftableitung (6) zum Verbraucher.
